# EUROPÄISCHE PATENTSCHRIFT

(11) **EP 3 481 275 B1**
(45) Veröffentlichungstag und Bekanntmachung des Hinweises auf die Patenterteilung: **02.09.2020**
(21) Anmeldenummer: 17735493.3
(22) Anmeldetag: 04.07.2017
(51) Int. Cl.: A61B 5/00, A61B 5/103, A61Q 5/00

(54) **VERFAHREN UND VORRICHTUNG ZUM ERMITTELN EINES SCHÄDIGUNGSGRADS VON HAAR UND VERFAHREN ZUM ERMITTELN EINES NUTZERSPEZIFISCHEN MITTELS ZUR HAARBEHANDLUNG**
METHOD AND DEVICE FOR ASCERTAINING A DEGREE OF DAMAGE TO HAIR, AND METHOD FOR ASCERTAINING A USER-SPECIFIC AGENT FOR TREATING HAIR
PROCÉDÉ ET DISPOSITIF POUR DÉTERMINER UN DEGRÉ DE DÉTÉRIORATION DES CHEVEUX ET PROCÉDÉ POUR DÉTERMINER UN MOYEN POUR TRAITER LES CHEVEUX SPÉCIFIQUE À L'UTILISATEUR

(30) Priorität: 05.07.2016 DE 102016212202
(43) Veröffentlichungstag der Anmeldung: 15.05.2019
(73) Patentinhaber: Henkel AG & Co. KGaA, 40589 Düsseldorf (DE)
(72) Erfinder: KNÜBEL, Georg, 40219 Düsseldorf (DE); VOSS-KLIMKE, Meike, 40764 Langenfeld (DE); SEILER, Oliver, 40597 Düsseldorf (DE); BÜTFERING, Ludger, 52385 Nideggen-Rath (DE); KROOS, Astrid, 40789 Monheim (DE); KOENEN, Annika, 41516 Grevenbroich (DE)
(86) Internationale Anmeldenummer: PCT/EP2017/066572
(87) Internationale Veröffentlichungsnummer: WO 2018/007353

(56) Entgegenhaltungen:
- WO-A1-2015/166403
- US-A1- 2002 010 556
- US-A1- 2006 281 994
- KOJI TAKADA ET AL: "Influence of Oxidative and/or Reductive Treatment on Human Hair (I): Analysis of Hair-Damage after Oxidative and/or Reductive Treatment", JOURNAL OF OLEO SCIENCE, Bd. 52, Nr. 10, 1. Januar 2003 (2003-01-01), Seiten 541-548, XP055002836, ISSN: 1345-8957, DOI: 10.5650/jos.52.541

## Beschreibung

Die Erfindung betrifft ein Verfahren und eine Vorrichtung zum Ermitteln eines Schädigungsgrads von Haar und ein Verfahren zum Ermitteln eines nutzerspezifischen Mittels zur Haarbehandlung.

Bei einer Behandlung von Haar mit kosmetischen Produkten kann eine Wirkung des Produkts, z.B. eine Intensität einer Färbung, stark vom Schädigungsgrad des Haars abhängen.

Deshalb kann eine Ermittlung einer Schädigung des Haars von großer Bedeutung sein.

Haar kann durch natürliche oder künstlich herbeigeführte Vorgänge geschädigt werden. Der wichtigste Schädigungstyp kann dabei eine oxidative Schädigung sein.

Die natürlichen Vorgänge können beispielsweise eine kombinierte (z.B. gleichzeitige) Einwirkung von UV-Licht und Sauerstoff (O₂) auf das Haar aufweisen.

Die künstlich herbeigeführten Vorgänge können dabei beispielsweise ein Anwenden von Haarfärbemitteln (auch als Colorationen bezeichnet), ein Blondieren, und/oder ein Erzeugen einer Dauerwelle aufweisen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Der Schädigungsvorgang kann dabei ursächlich durch eine Oxidation von Aminosäuren, beispielsweise eine Oxidation der im Haar sehr häufig vorkommenden Aminosäuren Cystin und Cystein zu Cysteinsäure, erfolgen. Cystin kann im Haar intermolekulare Disulfidbrücken (auch als S-S-Brücken bezeichnet) ausbilden, so dass das Cystin äußerst wichtig ist für die mechanische Stabilität des Haars.

Die Oxidation dieser Brücken zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenrauhigkeit, negativ beeinflusst werden. Auch Ergebnisse kosmetischer Behandlungen, insbesondere schädigender Verfahren, können bereits in einem frühen Schädigungsstadium massiv verändert sein im Vergleich zu einem Ergebnis bei ungeschädigtem Haar.
Der beschriebene Schädigungsmechanismus ermöglicht es, den Grad der wichtigsten, nämlich der oxidativen Schädigung, durch eine Bestimmung eines Gehaltes an Cysteinsäure exakt zu ermitteln. In einem akademischen und industriellen Bereich können einem Forscher bzw. Entwickler eine Vielzahl von physikalischen und chemisch-analytischen Verfahren zur Verfügung stehen, um eine Ermittlung des Schädigungsgrads durchzuführen, beispielsweise eine quantitative Ermittlung des oxidativen Schädigungsgrads.
Herkömmlich werden hierbei chromatographische Verfahren verwendet, wie beispielsweise Hochleistungsflüssigkeitschromatographie (HPLC) nach einem aufwändigen sauren hydrolytischen Aufschluss der Haarprobe.
Ungeschädigtes Haar kann typischerweise einen Cysteinsäuregehalt in einem Bereich von etwa 0.5% bis etwa 1% (nach Gewicht) aufweisen. Bei Vorliegen einer Schädigung, beispielsweise infolge mehrfachen Ultrablondierens und/oder anderer Schädigungsmechanismen, kann der Cysteinsäuregehalt auf über 15% (Gew.) ansteigen.
Allerdings sind all diese chromatographischen Verfahren kompliziert und apparativ aufwändig, so dass sie einem Endverbraucher nicht zur Verfügung stehen.
Die Dokumente US2006/281994 A1 und US 2002/010556 A1 offenbaren Verfahren zur Bestimmung der Haarschädigung. Das Dokument WO 2015/166403 A1 beschreibt ein Verfahren zur Bestimmung der Zusammensetzung eines Haarfärbemittels. Nicht-Patentliteratur "Influence of Oxidative and/or reductive Treatment on Human Hair (I): Analysis of Hair-damage after Oxidative and/or Reductive Treatment" von Koii Takada et al., Journal of Oleo Science Bd. 52. Nr. 10. Seiten 541-548 beschreibt Experimente an Haarproben.
Schädigende kosmetische Behandlungen, beispielsweise Haarcolorationen, Hitzeanwendungen, Dauerwellen oder oxidative Prozeduren wie z.B. Blondieren, und viele andere mehr, werden typischerweise im privaten Bereich oder im Bereich kommerzieller Dienstleistungen am Endverbraucher ausgeführt. Obwohl eine Durchführung eines weiteren schädigenden Verfahrens an vorgeschädigtem Haar zu katastrophalen Ergebnissen bis hin zu einem vollständigen Haarbruch führen kann, bestand in diesem Rahmen bisher keine Möglichkeit, den Grad der Vorschädigung des Haars, beispielsweise quantitativ, zu bestimmen.
In verschiedenen Ausführungsbeispielen werden ein in der Nutzung einfaches Verfahren und eine entsprechende Vorrichtung bereitgestellt, welche mit Hilfe von NIR-Spektroskopie und/oder IR-Spektroskopie und multivariaten Kalibrierverfahren eine präzise Bestimmung eines Grades oxidativer Schädigung von Haar ermöglicht.

In verschiedenen Ausführungsbeispielen kann aufgrund einer einfachen experimentellen Durchführung durch Nutzung neuartiger miniaturisierter NIR-Sensoren eine mobile

Datenverarbeitungsvorrichtung, z.B. ein Smartphone, ein Tablet oder ein Laptop, für die Vorrichtung und das Verfahren genutzt werden.

In verschiedenen Ausführungsbeispielen wird ferner ein Verfahren bereitgestellt, um basierend auf Ergebnissen des Verfahrens zum Ermitteln des Schädigungsgrads von Haar geeignete, ggf. sogar personalisierte Kosmetika bereitzustellen. Dabei kann das Verfahren auch bei der Personalisierung geeignet sein, in großer Zahl ausgeführt zu werden (so genannte "mass customization" bzw. "mass customized").

In verschiedenen Ausführungsbeispielen kann ein Nahinfrarot- (NIR-) und/oder ein Infrarot- (IR-)Spektrum gewonnen werden, beispielsweise mittels ATR-(Nah-)Infrarotspektroskopie (von Englisch "attenuated total reflection", auf Deutsch "abgeschwächte Totalreflexion"). Durch eine Anwendung von mathematischen Modellen kann mittels Vermessung von Kalibrier-Haarproben, welche einen anhand eines bekannten analytischen Verfahrens ermittelten Cysteinsäuregehalt aufweisen, ein mathematisches Modell erstellt werden, welches dann bei einer Haarprobe, auch als Tresse bezeichnet, des Verbrauchers anhand des aufgenommenen NIR- bzw. IR-Spektrums eine Berechnung eines Gehalts an Cysteinsäure, und damit der Haarschädigung, erlaubt. Eine Analyse des Spektrums und eine Anwendung des Modells kann dabei beispielsweise (mit geeigneten Apps) mittels bekannter Smartphones, Tablets o.ä. ausgeführt werden.

Erfindungsgemäß wird ein Verfahren zum Ermitteln eines Schädigungsgrads von Haar bereitgestellt. Das Verfahren weist auf: während eines Belichtens einer Haarprobe des Haars mit Nahinfrarot- und/oder Infrarotlicht, Aufnehmen eines Spektrums von zumindest einem Teil des Nahinfrarot- und/oder Infrarotlichts, welches von der Haarprobe reflektiert und/oder gestreut wird, Vergleichen zumindest eines Teils des Spektrums mit einem mittels Nahinfrarot- und/oder Infrarotspektren und Schädigungsgraden einer Mehrzahl von Kalibrierhaarproben gewonnenen spektroskopischen Kalibriermodell, und Ermitteln des Schädigungsgrads des Haars unter Einbeziehung des Vergleichs.

Erfindungsgemäß weist das Verfahren ferner ein Erstellen des Kalibriermodells auf, wobei das Erstellen des Kalibriermodells aufweist: für die Mehrzahl von Kalibrierhaarproben ein Aufnehmen eines Kalibrierspektrums von zumindest einem Teil des Nahinfrarot- und/oder Infrarotlichts, welches von der Kalibrierhaarprobe reflektiert und/oder gestreut wird während eines Belichtens der Kalibrierhaarprobe mit Nahinfrarot- und/oder Infrarotlicht, ein Ermitteln eines Schädigungsgrads der Kalibrierhaarprobe mittels eines unabhängigen analytischen Verfahrens, und ein Zuweisen des Schädigungsgrads zum Kalibrierspektrum, und ein Ermitteln einer Korrelation zwischen der Mehrzahl von Kalibrierspektren und der Mehrzahl von Schädigungsgraden.

Erfindungsgemäß weist das analytische Verfahren ein Ermitteln eines Cysteinsäuregehalts auf.
In verschiedenen Ausführungsbeispielen kann das Ermitteln des Cysteinsäuregehalts ein chromatographisches Verfahren aufweisen.
In verschiedenen Ausführungsbeispielen kann das Ermitteln einer Korrelation zwischen der Mehrzahl von Kalibrierspektren und der Mehrzahl von Schädigungsgraden ein Anwenden eines Partial Least Squares-Algorithmus aufweisen.
In verschiedenen Ausführungsbeispielen kann der zumindest eine Teil des Nahinfrarot- und/oder Infrarotlichts einen (Nahinfrarot-)Wellenzahlbereich von 5022 cm⁻¹ bis 4020 cm⁻¹ aufweisen.
In verschiedenen Ausführungsbeispielen kann der zumindest eine Teil des Nahinfrarot- und/oder Infrarotlichts einen (Infrarot-)Wellenzahlbereich um etwa 1040 cm⁻¹ aufweisen.
In verschiedenen Ausführungsbeispielen kann der zumindest eine Teil des Nahinfrarot- und/oder Infrarotlichts einen (Infrarot-)Wellenzahlbereich von 12.820 bis 7692 cm⁻¹ aufweisen.

Erfindungsgemäß wird eine Vorrichtung zum Ermitteln eines Schädigungsgrads von Haar bereitgestellt. Die Vorrichtung kann eine NIR-Lichtquelle oder/und eine IR-Lichtquelle zum Belichten einer Haarprobe des Haars mit NIR- bzw. IR-Licht aufweisen, ein Spektrometer zum Aufnehmen von zumindest einem Teil eines Spektrums von NIR- bzw. IR-Licht, welches mit der Haarprobe gewechselwirkt hat, und eine Datenverarbeitungsvorrichtung mit einem Datenspeicher, in welchem ein mittels einer Mehrzahl von Kalibrierhaarproben gewonnenes spektroskopisches Kalibriermodell gespeichert ist, und mit einem Prozessor zum Vergleichen zumindest eines Teils des Spektrums mit dem spektroskopischen Kalibriermodell und zum Ermitteln des Schädigungsgrads des Haars unter Einbeziehung des Vergleichs.
In verschiedenen Ausführungsbeispielen können die NIR- bzw. IR- Lichtquelle und das Spektrometer eine integrierte Einheit bilden.
In verschiedenen Ausführungsbeispielen kann der zumindest eine Teil des Nahinfrarot- und/oder Infrarotlichts einen (Nahinfrarot-)Wellenzahlbereich von 5022 cm⁻¹ bis 4020 cm⁻¹ aufweisen.
In verschiedenen Ausführungsbeispielen kann der zumindest eine Teil des Nahinfrarot- und/oder Infrarotlichts einen (Infrarot-)Wellenzahlbereich um etwa 1040 cm⁻¹ aufweisen.

In verschiedenen Ausführungsbeispielen können die integrierte Einheit und/oder die Datenverarbeitungsvorrichtung mobile Geräte sein.

In verschiedenen Ausführungsbeispielen wird ein Verfahren zum Ermitteln eines nutzerspezifischen Mittels zur Haarbehandlung bereitgestellt. Das Verfahren kann ein Ermitteln eines Schädigungsgrads von Haar eines Nutzers gemäß verschiedenen Ausführungsbeispielen, ein computergestütztes Ermitteln von mittels einer Mehrzahl von Mitteln zur Haarbehandlung erzielbaren Behandlungsergebnissen mittels prädiktiver Analytik unter Einbeziehung des ermittelten Schädigungsgrads, und ein Auswählen des nutzerspezifischen Mittels anhand der ermittelten Behandlungsergebnisse aufweisen.

Gemäß verschiedenen Ausführungsformen kann das nutzerspezifische Mittel zur Haarbehandlung ein Haarpflegeprodukt, ein Haarfärbeprodukt, ein Blondierungsprodukt oder ein Stylingprodukt (z.B. für eine Haarglättung oder eine Dauerwelle) aufweisen.

Ausführungsbeispiele der Erfindung sind in den Figuren dargestellt und werden im Folgenden näher erläutert.

Es zeigen
Figur 1 eine schematische Darstellung eines Verfahrens und einer Vorrichtung zum Ermitteln eines Schädigungsgrads von Haar gemäß verschiedenen Ausführungsbeispielen;
Figur 2A und 2B Nahinfrarot- (FIG. 2A) bzw. Infrarot- (FIG. 2B) Spektren von Cysteinsäure;
Figur 3 eine Tabelle mit Vorhersageergebnissen für einen Schädigungsgrad einer Mehrzahl von Haarproben, welche mittels eines Verfahrens gemäß einem Ausführungsbeispiel gewonnen wurden;
Figur 4 ein Ablaufdiagramm, welches ein Verfahren zum Ermitteln eines Schädigungsgrads von Haar gemäß verschiedenen Ausführungsbeispielen darstellt; und
Figur 5 ein Ablaufdiagramm, welches ein Verfahren zum Ermitteln eines nutzerspezifischen Mittels zur Haarbehandlung gemäß verschiedenen Ausführungsbeispielen darstellt.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

In der vorliegenden Beschreibung werden die Begriffe Prädiktive Analytik, Predictive Analytics, Big Data und Data Mining synonym verwendet.

In der vorliegenden Beschreibung wird für Licht mit einer Wellenzahl in einem Bereich von 12.820 bis 4000 cm⁻¹ der Begriff Nahinfrarot (NIR) verwendet, und für Licht mit einer Wellenzahl in einem Bereich von 3999 bis 400 cm⁻¹ der Begriff Infrarot (IR).

Ein hierin beschriebenes Verfahren zum Ermitteln eines Schädigungsgrads von Haar kann gemäß verschiedenen Ausführungsbeispielen entweder unter Nutzung eines Nahinfrarot-Bereichs ausgeführt werden, d.h. mittels Bestrahlens des Haars mit Nahinfrarotlicht und Spektralanalyse von zumindest einem Teil des NIR-Lichts, nachdem dieses mit dem Haar gewechselwirkt hat (anders ausgedrückt: in Wechselwirkung getreten ist), oder unter Nutzung eines Infrarot-Bereichs, d.h. mittels Bestrahlens des Haars mit Infrarotlicht und Spektralanalyse von zumindest einem Teil des IR-Lichts, nachdem dieses mit dem Haar gewechselwirkt hat, oder unter Verwendung von sowohl dem Nahinfrarot- als auch dem Infrarotbereich, d.h. mittels Bestrahlens des Haars mit Nahinfrarot- und Infrarotlicht und Spektralanalyse von zumindest einem Teil des NIR- und zumindest einem Teil des IR-Lichts, nachdem dieses mit dem Haar gewechselwirkt hat.

Eine traditionelle Methode für eine Bestimmung eines Cysteinsäuregehalts von Haar basiert auf einem chemischen Aufschluss des Haars und anschließender Chromatographie der freigesetzten Aminosäuren mittels Hochdruckflüssigchromatographie (HPLC, abgeleitet vom entsprechenden englischen Begriff High Pressure Liquid Chromatography).

Als Alternative wurden in den vergangenen Jahren Methoden der Nahinfrarotspektroskopie etabliert. Diese Methoden erlauben eine direkte, zerstörungsfreie Prüfung des Cysteinsäuregehaltes und anderer Bestandteile ohne eine aufwändige Probenvorbereitung und ohne das Haar durch die Analyse in seiner Struktur zu verändern oder zu zerstören.

Dies kann ein schnelleres Erzielen von Ergebnissen ermöglichen. Darüber hinaus kann es möglich sein, die Haare nach der Messung weiteren Behandlungen zu unterziehen, so dass an einer Haarsträhne Mehrfachanwendungen durchgeführt werden können.

Zudem kann es möglich sein, relativ leicht Messungen an verschiedenen Haarpositionen durchzuführen (z.B. kopfhautnahes und kopfhautentferntes Haar), beispielsweise auch direkt am Kopf, ohne die Haarprobe entnehmen zu müssen.

Die Nahinfrarotspektroskopie kann beispielsweise dann angewendet werden, wenn eine Probenaufbereitung schwierig oder unmöglich ist und in kurzer Zeit eine Vielzahl von Proben zu analysieren ist, z.B. in einer pharmazeutischen Produktion zur Chargenfreigabe oder für Messungen an Haut oder an anderen biologischen Objekten.

Die Nahinfrarotspektroskopie basiert auf einer Messung spektraler Absorption durch Schwingungsanregungen chemischer Bindungen. Abhängig davon, um welche Art von Bindung es sich handelt, kann eine Absorption bei unterschiedlichen Wellenlängen stattfinden. In der Regel kann die Absorption dem Lambert-Beerschen Gesetz folgen und damit proportional zu einer Konzentration eines für die Absorption verantwortlichen Bestandteils sein. Überlagernde Absorptionen sind additiv, so dass eine Quantifizierung auf Basis der Spektren möglich ist.

Allerdings kann es sich bei diesen Absorptionen um Obertöne (z.B. im NIR-Bereich) oder Kombinationsschwingungen handeln. Eine Zuordnung einzelner Absorptionswellenlängen kann dadurch erschwert sein.

Aus diesem Grunde können zur Auswertung der Spektren und insbesondere zur quantitativen Evaluierung chemometrische Verfahren eingesetzt werden.

Die NIR Spektroskopie kann auch deshalb geeignet sein zur Messung an Haaren, weil mit der Nahinfrarotstrahlung nicht nur die Oberfläche der Haare analysiert wird, sondern wegen der kleinen Absorptionsquerschnitte für die nahinfrarote Strahlung (z.B verglichen mit Licht im sichtbaren Wellenlängenbereich) die Haare zumindest teilweise durchdrungen werden können.

Eine Auswertung der Spektren kann mittels eines Rechenmodells erfolgen, welches in einer Kalibrierphase und Validierphase anhand eines genügend großen Kollektivs von Vergleichsspektren entwickelt werden kann. Dazu kann es erforderlich sein, dass für alle Spektren Referenzwerte der zu kalibrierenden Kenngrößen vorliegen. Im Falle der Cysteinsäureanalytik an Haaren können diese Werte mittels des traditionellen Verfahrens der HPLC gewonnen werden.

Das Rechenmodell kann ein künstliches System sein, das beispielsweise aus den Kalibrier-Haarproben lernt und diese nach Beendigung der Lernphase verallgemeinern kann. Das heißt, es werden nicht einfach die Beispiele auswendig gelernt, sondern es werden Muster und Gesetzmäßigkeiten in den Lerndaten erkannt. Hierzu können unterschiedliche Ansätze verfolgt werden. Beispielsweise kann ein überwachtes Lernen, ein teilüberwachtes Lernen, ein unüberwachtes Lernen, ein bestärktes Lernen und/oder ein aktives Lernen eingesetzt werden insbesondere in Verbindung mit *deep* learning-Verfahren. Ein überwachtes Lernen kann beispielsweise mittels eines künstlichen neuronalen Netzes (etwa einem rekurrenten neuronalen Netz) oder mittels einer Support Vector Machine erfolgen. Auch ein unüberwachtes Lernen kann beispielsweise mittels eines künstlichen neuronales Netzes (beispielsweis eines Autoencoders) erfolgen.

In verschiedenen Ausführungsbeispielen kann ein vermessener Nahinfrarot (NIR)-Bereich Wellenzahlen von etwa 12.820 cm⁻¹ bis etwa 4000 cm⁻¹ aufweisen. Dieser Wellenlängenbereich kann u.a. charakteristische Oberton- und Kombinationsschwingungen von z.B. CH-, OH- und NH-Gruppen aufweisen.

In verschiedenen Ausführungsbeispielen können NIR Spektren von Cystin im Wellenzahlbereich von etwa 6200 cm⁻¹ bis etwa 5500 cm⁻¹ charakteristische Absorptionsbanden zeigen. Verändert sich das Haar z.B. durch stärker werdende Schädigung (Zunahme des Cysteinsäuregehaltes), kann sich das im NIR-Spektrum auf die für die Cysteinsäure charakteristischen Banden bei 5022 cm⁻¹ bis 4020 cm⁻¹ auswirken.

Eine chemometrische Analyse der gemessenen (N)IR-Spektren kann in verschiedenen Ausführungsbeispielen in einem NIR-Wellenzahlbereich von etwa 8000 cm⁻¹ bis etwa 4020 cm⁻¹ (bei einer Auflösung von beispielsweise 1 cm⁻¹) oder/und in einem IR-Wellenzahlbereich von etwa 1100 cm⁻¹ bis etwa 1000 cm⁻¹ (bei einer Auflösung von beispielsweise 1 cm⁻¹) erfolgen. Hier können sich u. a. die relevanten Absorptionsbanden der zu analysierenden Komponente Cysteinsäure befinden.

Neben der direkten Bestimmung des Cysteinsäuregehalts über die charakteristischen Absorptionsbanden der Cysteinsäure, insbesondere im Bereich von 5022 cm⁻¹ bis 4020 cm⁻¹, kann auch eine indirekte Bestimmung des Cysteinsäuregehalts erfolgen. Die indirekte Bestimmung des Cysteinsäuregehalts erfolgt in einem Wellenzahlenbereich, in dem Cysteinsäure keine charakteristischen oder relevanten Absorptionsbanden aufweist.

Zwischen dem Gehalt an Cysteinsäure und dem Gehalt an Melanin im Haar besteht eine inverse Korrelation, die indirekt - über die Bestimmung des Melaningehaltes - die Bestimmung des Gehalts an Cysteinsäure ermöglicht. Es wurde insbesondere gefunden, dass der Prozess der oxidativen Schädigung (Bildung von Cysteinsäure) beim Blondieren oder Färben von Haaren mit dem Abbau von Melanin verknüpft ist.

Das in den Haaren vorkommende Melanin absorbiert nicht nur im sichtbaren Spektrum (VIS), sondern auch im kurzwelligen Nahinfrarot-Spektrum, d.h. bis etwa 1300 nm. Ohne an diese Theorie gebunden sein zu wollen, wird vermutet, dass es zusätzlich zur Bildung spezifischer Oxidationsprodukte des Melanins kommt, die auch noch Absorptionen im kurzwelligen NIR-Bereich aufweisen.

Es hat sich gezeigt, dass sich auch mit Hilfe von kurzwelligen Nahinfrarotspektren mit einem Wellenzahlbereich von 12.820 bis 7692 cm⁻¹, also in einem Wellenzahlbereich in dem Cysteinsäure keine charakteristische Absorption zeigt, zuverlässige Kalibriermodelle erstellen lassen, die eine Korrelation zwischen dem kurzwelligen Nahinfrarotspektrum und dem Cysteingehalt von Haarproben herstellen und die im Wesentlichen dieselbe Qualität aufweisen, wie Auswertungen über den gesamten oder über längerwellige Teile des Nahinfrarot- und/oder Infrarotspektralbereich, insbesondere längerweillige Bereiche mit charakteristischen Absorptionen der Cysteinsäure.

Zusammengefasst heißt das, dass der Gehalt an Cysteinsäure und somit die Bestimmung eines Schädigungsgrads von Keratinfaser indirekt über den Gehalt an Melanin und ggf. der Oxidationsprodukte von Melanin in den Keratinfasern bestimmt werden kann.

In verschiedenen Ausführungsbeispielen kann ein vermessener Nahinfrarot (NIR)-Bereich Wellenzahlen von etwa 12.820 bis 7692 cm⁻¹ aufweisen.

In verschiedenen Ausführungsbeispielen kann anhand von Ergebnissen einer quantitativen rechnergestützten Auswertung (auch als chemometrischen Analyse bezeichnet) für eine Mehrzahl von Kalibrationshaarproben in Kombination mit mittels eines unabhängigen Verfahrens, z.B. mittels HPLC, für dieselben Kalibrationshaarproben gewonnenen Werten für einen Cysteinsäuregehalt der jeweiligen Kalibrationshaarprobe, ein Kalibrationsmodell erstellt werden. Dies kann auch als Kalibrationsphase des Verfahrens zum Ermitteln eines Schädigungsgrads von Haar bezeichnet werden.

Liegt das Kalibrationsmodell vor, kann in verschiedenen Ausführungsbeispielen sehr einfach anhand eines für eine zu messende Haarprobe (für eine Unterscheidung von den Kalibrationshaarproben auch als Messhaarprobe bezeichnet, das zugehörige Spektrum als Messspektrum) gewonnenen (N)IR-Spektrums die Konzentration der Cysteinsäure (als Maß für die Haarschädigung) aus den Spektren im Vergleich mit den Kalibrationsspektren berechnet werden. Dabei kann das Kalibrationsmodell die Berechnung der Cysteinsäurekonzentration sowohl dann erlauben, wenn das Messspektrum im Wesentlichen einem der Kalibrationsspektren entspricht (bzw. eine jeweils aus den Spektren ermittelte Größe, beispielsweise ein Wert für die Absorption im für die Cysteinsäure charakteristischen Wellenlängenbereich, beispielsweise eine Äquivalentbreite oder Ähnliches, im Wesentlichen identisch sind), als auch dann, wenn das Messspektrum bzw. die daraus ermittelte Größe zwischen zwei Kalibrationsspektren oder jenseits eines Kalibrationsspektrums einzuordnen wäre. Anders ausgedrückt kann das Kalibrationsmodell anhand der diskreten Kalibrationsspektren und der dazugehörigen analytisch bestimmten Cysteinsäurekonzentrationswerte als kontinuierliches Modell gebildet werden, welches eine Inter- und Extrapolation der diskreten Datenpunkte ermöglicht.

In verschiedenen Ausführungsbeispielen kann die Modellbildung, d.h. eine quantitative Auswertung der Kalibrationsspektren und der zugehörigen Cysteinsäurekonzentrationswerte, nach der so genannten "Partial Least Square"-Methode (PLS) ausgeführt werden. Beispielsweise kann bei der Erstellung des Kalibrationsmodells mit Hilfe eines Partial Least Squares (PLS)-Algorithmus eine Korrelation zwischen den spektroskopischen Daten und den entsprechenden Konzentrationswerten der einzelnen Komponenten (hier also beispielsweise der Cysteinsäure, sofern weitere Haarbestandteile alternativ oder zusätzlich einen Rückschluss auf den Schädigungsgrad des Haars erlauben und charakteristische Absorptionsmerkmale im NIR- oder IR-Spektrum zeigen, können diese alternativ oder zusätzlich zur Cysteinsäure herangezogen werden) berechnet werden.

Je umfangreicher eine Datenbasis ist (d.h. je mehr Spektren und zugehörige Referenzwerte bereitgestellt werden), umso besser kann eine Vorhersagegenauigkeit des Kalibrationsmodells sein. Als Referenzwerte sind hier die Werte bezeichnet, die für die Kalibrationshaarproben in einem Labor über ein anderes unabhängiges analytisches Verfahren (hier beispielsweise die HPLC nach Aufschluss zum Ermitteln der Cysteinsäurekonzentration) quantitativ worden sein können.

Im Rahmen der Modellbildung können gemäß verschiedenen Ausführungsbeispielen aus der Korrelation von Spektren und Referenzwerten virtuelle Spektren - sogenannte Faktoren - ermittelt werden, welche die wesentlichen, komponentenbezogenen spektralen Anteile wiedergeben können.

Mit diesen Faktoren können in verschiedenen Ausführungsbeispielen dann die vorherzusagenden Spektren optimal zusammengesetzt (gefittet) werden. Die dabei zu einem optimalen Ergebnis führenden Wichtungsfaktoren der einzelnen Faktoren können eine Grundlage der quantitativen Ergebnisberechnung bilden.

Folgende Parameter können gemäß verschiedenen Ausführungsbeispielen zur Optimierung des PLS-Modells dienen: ein zur Auswertung genutzter Frequenzbereich, eine mathematische Datenvorbehandlung, eine Auswahl der optimalen Faktorenzahl, und/oder eine Eliminierung von Ausreißern (spektrale und/oder Konzentrationsausreißer).

In verschiedenen Ausführungsbeispielen kann eine Qualität des entwickelten Kalibrationsmodells anhand mindestens eines statistischen Leistungsparameters bewertet werden. Der mindestens eine statistische Leistungsparameter kann beispielsweise ein Korrelationskoeffizient R² (auch als Bestimmtheitsmaß bezeichnet) und/oder ein Analysenfehler einer Kreuzvalidierung RMSEP (abgeleitet vom englischen Begriff "Root Mean Square Error of Prediction") sein.

In verschiedenen Ausführungsbeispielen können für das Entwickeln des Kalibrationsmodells eine Mehrzahl von Modellen erstellt werden, welche im Rahmen einer Optimierung jeweils validiert werden können, so dass das Modell, welches die besten Vorhersageergebnisse ermöglicht, als das Kalibrationsmodell bestimmt werden kann. Dazu können beispielsweise zwei verschiedene Verfahren, eine rechnergestützte Kreuzvalidierung und/oder eine Validierung mit externen Referenzproben, eingesetzt werden.

Bei einer Kreuzvalidierung können Testkalibrationen von einem Probenset berechnet werden, denen jeweils eine der Proben entnommen wurde. Mit dieser Subkalibrierung kann dann jeweils die herausgenommene Probe berechnet werden. Führt man dieses Prozedere nun für alle Proben nacheinander durch, so kann man einen validen Ergebnissatz von dessen Einzelproben erhalten, deren Ergebnisse betrachtet werden, und welche nicht in den zugrundeliegenden Kalibriersatz eingeflossen sind. Anhand der Ergebnisse dieser Kreuzvalidierung können die Parameter des jeweiligen Optimierungsschrittes bewertet werden.

Alternativ oder ergänzend kann die Validierung an einem unabhängigen Validierungsdatensatz durchgeführt werden. Dazu kann eine Mehrzahl von unabhängigen Validierproben mit bekannter Analytkonzentration (also beispielsweise mit bekanntem Cysteinsäuregehalt) mit Hilfe des chemometrischen Kalibriermodells vorhersagt werden. Unabhängig heißt hier, dass die Validierproben nicht im Kalibrierdatensatz enthalten sind. Als ein Nachteil dieser Methode kann betrachtet werden, dass die wertvollen Validierproben nicht zur Verbesserung der Kalibration genutzt werden können.

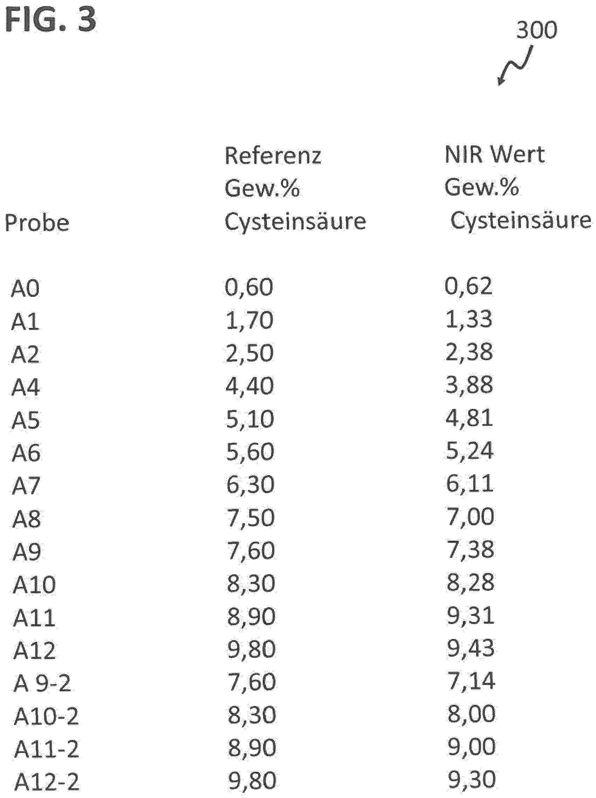

Für beispielhafte Ergebnisse einer Ermittlung eines Schädigungsgrads von Haar gemäß einem Ausführungsbeispiel und eine zugehörige Validierung siehe die Tabelle in FIG. 3 und die zugehörige Beschreibung.

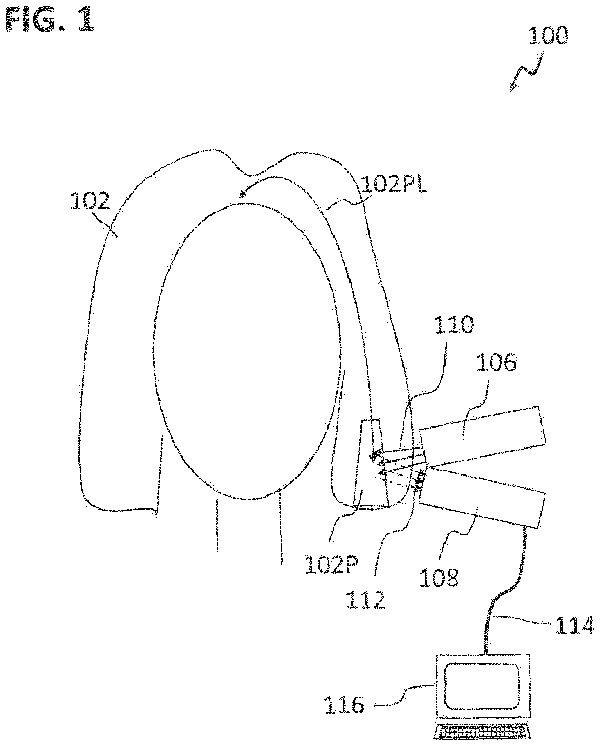

FIG. 1 zeigt in einer Ansicht 100 eine schematische Darstellung eines Verfahrens zum Ermitteln eines Schädigungsgrads von Haar gemäß verschiedenen Ausführungsbeispielen.

Gemäß verschiedenen Ausführungsbeispielen kann zum Ermitteln eines Schädigungsgrads von Haar 102 eines Nutzers eine Haarprobe 102P untersucht werden. Die Haarprobe 102P kann dabei am Kopf belassen oder entfernt werden. Die Haarprobe 102P kann sich in einer Entfernung 102PL von einer Kopfhaut des Nutzers befinden bzw. von dort entnommen werden. Die Haarprobe 102P kann eine Mindestmenge von Haaren aufweisen, die beispielsweise ausgedrückt sein kann als eine minimale Fläche, welche flächendeckend mit der (z.B. flach ausgebreiteten) Haarprobe 102P abgedeckt sein kann, beispielsweise mindestens 1 cm², oder beispielsweise als ein Mindestgewicht, beispielsweise mindestens 0,5 g.

In verschiedenen Ausführungsbeispielen kann zum Ermitteln des Schädigungsgrads des Haars 102 eine Vorrichtung genutzt werden, wie sie in FIG. 1 beispielhaft schematisch in einer Ansicht 100 dargestellt ist.

In verschiedenen Ausführungsbeispielen kann die Haarprobe 102Pb so ausgebreitet werden, dass sie zumindest einen Wechselwirkungsbereich bedeckt, welcher von einer Lichtquelle 106 mit NIR- oder/und IR-Licht 110 beleuchtet wird und aus welchem das Licht 110, nach einer Wechselwirkung mit der Haarprobe 102 als zu analysierendes Licht 112 bezeichnet, in ein Spektrometer 108 eintritt.

In verschiedenen Ausführungsbeispielen kann die Haarprobe 102P mit NIR-Licht 110 und/oder mit IR-Licht 110 beleuchtet werden. Das NIR-Licht 110 kann einen Wellenzahlbereich von etwa 12820 cm⁻¹ bis etwa 4000 cm⁻¹ abdecken, oder mindestens einen geeigneten Teilbereich daraus, beispielsweise von etwa 8000 cm⁻¹ bis etwa 4020 cm⁻¹, beispielsweise von etwa 5022 cm⁻¹ bis etwa 4020 cm⁻¹. Ein weiterer bevorzugter Teilbereich deckt Wellenzahlen von etwa 12.820 cm⁻¹ bis 9090 cm⁻¹ und besonders bevorzugt von etwa 12500 cm⁻¹ bis 9524 cm⁻¹ ab. Das IR-Licht 110 kann einen Wellenzahlbereich von etwa 3999 cm⁻¹ bis etwa 400 cm⁻¹ abdecken, oder mindestens einen geeigneten Teilbereich daraus, beispielsweise von etwa 3000 cm⁻¹ bis etwa 500 cm⁻¹, beispielsweise von etwa 1100 cm⁻¹ bis etwa 1000 cm⁻¹.

In verschiedenen Ausführungsbeispielen kann eine Lichtquelle 106 des NIR- bzw. des IR-Lichts 110 beispielsweise eine NIR-Lampe oder/und eine IR-Lampe sein, oder jede andere herkömmliche Lichtquelle, die ein geeignetes Lichtspektrum bereitstellt.

In verschiedenen Ausführungsbeispielen, z.B. wenn eine ATR-Spektroskopie genutzt wird, kann das von der Lichtquelle 106 emittierte Licht 110 in einem Medium geführt werden, in welchem es (z.B. mehrfach) total reflektiert wird. Die Haarprobe 102P kann dabei so nahe an das Medium herangebracht werden, beispielsweise in körperlichen Kontakt mit dem Medium, dass sie mit evaneszentem Licht des Lichts 110 wechselwirken kann, beispielsweise spektral charakteristische Anteile des Lichts 110 absorbieren kann.

In verschiedenen Ausführungsbeispielen können andere NIR- und/oder IR-Spektroskopieverfahren genutzt werden, beispielsweise eine Diffuse-Reflexion-Spektroskopie (DRS).

In verschiedenen Ausführungsbeispielen kann bei dem Verfahren zum Ermitteln eines Schädigungsgrads von Haar, welches angewendet werden kann, ohne die Haarprobe zu zerstören (und beispielsweise sogar bei am Kopf des Nutzers belassenem Haar ausgeführt werden kann), mittels der (N)IR-Strahlung nur ein Oberflächenbereich des Haars erreicht werden. Eine Eindringtiefe von (N)IR-Strahlung in Haar kann typischerweise etwa bis zu 10 µm aufweisen, wohingegen ein typischer Haardurchmesser etwa 60 bis 70 µm aufweisen kann. Allerdings können mittels der unabhängigen Analyse des Cysteinsäuregehalts der Haarproben, welche für die Bildung des Kalibrationsmodells genutzt wird, die Oberflächeneigenschaften der Kalibrationshaarproben mit dem Maß für den Schädigungsgrad des Haars (hier also beispielsweise dem Cysteinsäuregehalt) korreliert werden, so dass für die Messhaarproben eine Vorhersage des Schädigungsgrads des gesamten Haars anhand des (N)IR-Spektrums ermöglicht ist, auch wenn das (N)IR-Spektrum nur eine Eigenschaft der Haaroberfläche widerspiegelt.

Dank einer fortschreitenden Verbesserung von (N)IR-Spektroskopievorrichtungen können diese in miniaturisierter, z.B. mobiler, Form bereitgestellt werden, beispielsweise als eine Einheit, welche die Lichtquelle 106 und das Spektrometer 108 als integrierte Bestandteile aufweisen kann.

Die (N)IR-Spektroskopievorrichtungen können beispielsweise in Form von Hand-Spektroskopievorrichtungen ("handheld") oder Aufsatz-Spektroskopievorrichtungen bereitgestellt werden.

Ein Beispiel für eine geeignete Hand-Spektroskopievorrichtung ist das "MicroNIR OnSite" der Firma Viavi Solutions Inc. Diese Spektroskopievorrichtung wird von einem Tablet oder einem Laptop über einen USB-Anschluss mit Strom versorgt und gesteuert und ermöglicht mit einer Messzeit von zwischen 0,25 und 0,5 Sekunden die Aufnahme der Nahinfrarot- und/oder Infrarotspektren der Keratinfasern eines Individuums in Echtzeit. Die Spektroskopievorrichtung weist zwei integrierte Vakuum-Wolfram-Lampen als Lichtquelle und ein InGaAs-Photodioden-Array mit 128 Pixeln auf. Das "MicroNIR OnSite" arbeitet in einem Wellenzahlenbereich von 6060 bis 10526 cm⁻¹. Der Abstand zwischen den Keratinfasern und dem Glas der Handspektroskopievorrichtung kann zwischen 0 und 15 mm betragen, wobei ein Abstand von 3 mm bevorzugt ist.

In einer Ausführungsform der Erfindung wird das ganze Verfahren zur Ermittlung einer individualisierten Haarbehandlung durch das Tablet oder den Laptop durchgeführt, welcher die "MicroNIR OnSite"-Spektroskopievorrichtung mit Strom versorgt und steuert. Alternativ können die gewonnen spektroskopischen Daten an eine weitere (mobile) Datenverarbeitungsvorrichtung, insbesondere ein weiteres smartes Endgerät, gesendet werden, welches dann das Verfahren zur Ermittlung einer individualisierten Haarbehandlung durchführt. Die Übertragung der spektroskopischen Daten kann beispielsweise kabellos mittels WLAN (WiFi) oder Bluetooth erfolgen.

Eine weitere geeignete Hand-Spektroskopievorrichtung ist das "i-Spec Nano" der Firma B&W Tek. Die Spektroskopievorrichtung wird über einen USB-Anschluss und einer daran angeschlossenen (mobilen) Datenverarbeitungsvorrichtung oder über eine Batterie mit Strom versorgt. Die Spektroskopievorrichtung weist eine Lichtquelle auf und arbeitet in einem Wellenzahlenbereich von 4545 bis 7692 cm⁻¹. Die Übertragung der spektroskopischen Daten an eine (mobile) Datenverarbeitungsvorrichtung, welche dann das Verfahren zur Ermittlung einer individualisierten Haarbehandlung durchführt, kann kabellos mittels WLAN (WiFi) oder Bluetooth erfolgen.

Ebenfalls geeignet ist die Hand-Spektroskopievorrichtung "QualitySpec Trek" der Firma ASD Inc. Dieses arbeitet in einem Wellenzahlenbereich von 28571 bis 400 cm⁻¹.

Eine weitere geeignete Hand-Spektroskopievorrichtung ist das "SCiO by Consumer Physics", welche mit Hilfe der integrierten App "SpectroScan" die spektroskopischen Daten auf einem smarten Endgerät anzeigt. Die Hand-Spektroskopievorrichtung arbeitet im kurzwelligen Bereich des NIR und zwar bei Wellenzahlen von 9090 bis 14285 cm⁻¹ (entspricht 700 bis 1100 nm). Die Auswertung der gemessenen Daten erfolgt mit Hilfe einer Cloud, in der beispielsweise eine Materialdatenbank, chemometrische Modelle und Algorithmen gespeichert sind.

Noch weitere geeignete Hand-Spektroskopievorrichtungen sind von der Firma Attonics Systems erhältlich, welche entweder in Wellenzahlenbereichen von 9090 bis 26.315 cm⁻¹ (VIS-NIR) oder von 3333 bis 10.000 cm⁻¹ (NIR) arbeiten. Diese Spektroskopievorrichtungen basieren auf Interferometern und weisen einen hohen Lichtdurchsatz und eine hohe spektrale Auflösung (< 5 nm für die VIS-NIR-Spektroskopievorrichtung und < 20 nm für die NIR-Spektroskopievorrichtung) auf. Die Spektroskopievorrichtungen weisen einen Multi-Phase Shift Array (MPA) Chip und eine optische Anordnung in einem kreisförmigen Rohr auf. Ferner sind die Spektroskopievorrichtungen kompatibel mit mobilen Datenverarbeitungsvorrichtungen.

Weitere Beispiele für VIS-NIR-Spektroskopievorrichtungen sind die Miniaturspektroskopievorrichtungen "USB2000-VIS-NIR" und "USB4000-VIS-NIR" der Firma Ocean Optics. Diese Spektroskopievorrichtungen arbeiten mit einem Wellenlängenbereich von 350 bis 1000 nm. Die Spektrometer werden über einen USB-Anschluss mit einer Datenverarbeitungsvorrichtung verbunden werden.

Eine weitere geeignete, miniaturisiertes NIR-Spektroskopievorrichtung befindet sich integriert in dem Smartphone H2 der Firma Changhong.

Daneben existieren eine Reihe von NIR-Sensoren oder NIR-Auswertungsmodule, welche in Hand-Spektroskopievorrichtungen verwendet werden können. Geeignete NIR-Auswertungsmodule sind die Module "DLP® NIRscan" und "DLP® NIRscan Nano" der Firma Texas Instruments. Diese weisen zwei Wolfram-Lampen und InGaAs-Photodioden als Detektoren auf. Das Modul "DLP® NIRscan" arbeitet im Wellenzahlenbereich von 4016 bis 7407 cm⁻¹ und das Modul "DLP® NIRscan Nano" im Bereich von 5882 bis 11111 cm⁻¹. Die Kommunikation der spektroskopischen Daten erfolgt kabellos über Bluetooth Low Energy. Mit Hilfe von "Software Developer Kits" (SDK), beispielsweise das Open Source SDK von KST Technologies, können Apps entwickelt werden, die die spektroskopischen Daten auswerten oder weiter verarbeiten.

Weitere geeignete NIR-Sensoren sind unter der Bezeichnung "NeoSpectra" von Si-Ware Systems erhältlich. Konkrete Sensoren umfassen: NeoSpectra SW62221-1.7, NeoSpectra SW62221-2.1 und NeoSpectra SW62221-2.5, welche in unterschiedlichen Wellenzahlenbereichen arbeiten.

In verschiedenen Ausführungsbeispielen kann das Spektrum an eine Datenverarbeitungsvorrichtung 116 übertragen werden. Dies ist mit dem Bezugszeichen 114 gekennzeichnet. Die Übertragung kann auf bekanntem Weg erfolgen, beispielsweise mittels eines Datenkabels, kabelloser Datenübertragung (z.B. Bluetooth, WLAN, Thread, ZigBee oder Near Field Communication (NFC)), oder eine Übertragung kann innerhalb einer Vorrichtung erfolgen, wenn das Spektrometer (und ggf. die Lichtquelle) Teil einer Datenverarbeitungsvorrichtung (z.B. Mobiltelefon, Tablet, Laptop) ist bzw. umgekehrt das Spektrometer 108 mit einer integrierten Datenverarbeitungsvorrichtung 116 gebildet ist.

Zum Empfangen und Weiterverarbeiten der Daten und für die Modellbildung kann die Datenverarbeitungsvorrichtung in verschiedenen Ausführungsbeispielen mit einer entsprechenden Software ausgestattet sein, beispielsweise einer App.

In verschiedenen Ausführungsbeispielen kann anhand des aufgenommenen (N)IR-Spektrums in Verbindung mit dem Kalibrationsmodell ein Cysteinsäuregehalt der Haarprobe wie oben beschrieben ermittelt werden.

In verschiedenen Ausführungsbeispielen kann der Schädigungsgrad in einer kategorialen Skala (z.B. leicht, mittel, schwer) ermittelt werden.

In verschiedenen Ausführungsbeispielen kann der Schädigungsgrad in einer metrischen Skala (z.B. Prozentanteil des Gehalts an Cysteinsäure) ermittelt werden.

In verschiedenen Ausführungsbeispielen kann das beschriebene Verfahren zum Ermitteln eines Schädigungsgrads von Haar mittels einer Datenverarbeitungsvorrichtung 116 ausgeführt werden.

Die Datenverarbeitungsvorrichtung kann, wie oben im Zusammenhang mit FIG. 1 beschrieben, beispielsweise eine mobile Datenverarbeitungsvorrichtung, beispielsweise ein Smartphone, ein Tablet oder einen Laptop, aber auch einen sonstigen Computer, wie einen Smart Mirror, aufweisen, oder jede andere Datenverarbeitungsvorrichtung, die geeignet ist, die Daten zu speichern und bereitzustellen, den Vergleich auszuführen und das Modell anzuwenden, ggf. auch das Modell zu erstellen, also beispielsweise jede Datenverarbeitungsvorrichtung mit hinreichend großem Datenspeicher und hinreichend leistungsfähigem Prozessor.

In verschiedenen Ausführungsbeispielen kann die Datenverarbeitungsvorrichtung ferner eingerichtet sein, in Abhängigkeit vom ermittelten Schädigungsgrad eine individuelle Haarbehandlungsanweisung zu ermitteln. Die individuelle Haarbehandlungsanweisung kann die Empfehlung von kommerziell erhältlichen Blondiermitteln und/oder Haarfärbemitteln und/oder Haarpflegeprodukten und/oder Haarstylingmitteln umfassen. Alternativ kann die individuelle Haarbehandlungsanweisung beispielsweise darin bestehen, die chemische Zusammensetzung eines Haarbehandlungsmittels, insbesondere eines Blondiermittels, eines Haarfärbemittels, eines Haarpflegeprodukts und/oder Haarstylingmittels zu ermitteln.

In verschiedenen Ausführungsbeispielen kann die Datenverarbeitungsvorrichtung mindestens eine Eingabevorrichtung zum Eingeben von Informationen in die Datenverarbeitungsvorrichtung aufweisen, beispielsweise zum Eingeben von Cysteinsäuregehalt-Messwerten für die Kalibrierung und ggf. zum Eingeben von Anweisungen, Parametern usw. für ein Ausführen des Verfahrens.

In verschiedenen Ausführungsbeispielen kann die Datenverarbeitungsvorrichtung mindestens eine Ausgabevorrichtung zum Ausgeben von Informationen aufweisen, beispielsweise zum Ausgeben von Ergebnissen des Verfahrens. Die Ausgabe der Ergebnisse kann graphisch und/oder akustisch erfolgen.

In verschiedenen Ausführungsbeispielen kann die mindestens eine Ausgabevorrichtung einen Bildschirm und/oder einen Drucker und/oder einen Lautsprecher aufweisen.

In verschiedenen Ausführungsbeispielen kann für eine Modellierung jedes beliebige Programm (z.B. eine App) genutzt werden, welches eine solche Funktionalität bereitstellt.

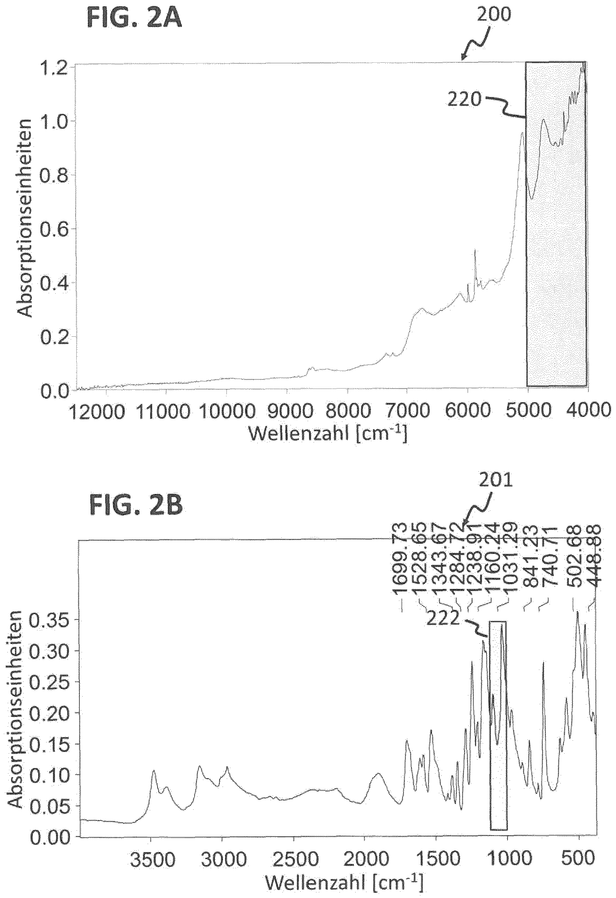

FIG. 2A zeigt ein NIR-Spektrum 200 von Cysteinsäure, FIG. 2B ein IR-Spektrum 201 von Cysteinsäure, jeweils als Absorptionseinheiten in Abhängigkeit von der Wellenzahl.

Mittels Boxen 220, 222 sind im NIR-Spektrum 200 in FIG. 2A (Box 220) und im IR-Spektrum 201 in FIG. 2B jeweils Spektralbereiche hervorgehoben, die für Cysteinsäure charakteristische Absorptionsmerkmale aufweisen können. Beispielhaft ist in FIG. 2A der Bereich von etwa 5022 cm⁻¹ bis etwa 4020 cm⁻¹ hervorgehoben, in FIG. 2B der Bereich von etwa 1100 cm⁻¹ bis etwa 1000 cm⁻¹.

Diese Spektralbereiche können in verschiedenen Ausführungsbeispielen für das Verfahren zum Ermitteln eines Schädigungsgrads von Haar genutzt werden.

Alternativ oder zusätzlich können andere bzw. weitere NIR- und/oder IR-Spektralbereiche genutzt werden, welche für die Cysteinsäure charakteristische Absorptionsmerkmale aufweisen.

FIG. 3 zeigt eine Tabelle 300 mit Vorhersageergebnissen für einen Schädigungsgrad von einer Mehrzahl von Haarproben (als Cysteinsäuregehalt in Gewichtsprozent), welche mittels eines Verfahrens gemäß einem Ausführungsbeispiel gewonnen wurden.

Für eine Beurteilung einer Qualität des Kalibrationsmodells, welches genutzt wurde, um die in der Tabelle 300 in der dritten Spalte wiedergegebenen Vorhersageergebnisse zu ermitteln, wurden eine Kreuzvalidierung und eine Validierung an einem unabhängigen Validierungsdatensatz ausgeführt.

Die Erstellung von Kalibrierungen für die Quantifizierung von Cysteinsäure wurde in diesem Beispiel im Wesentlichen basierend auf Kerling Haarmustern durchgeführt, die vor und nach verschiedenen Behandlungen vermessen wurden.

Um eine genügend große Datenbasis zu erhalten, wurden über einen größeren Zeitraum Proben gesammelt, die Spektren vermessen und dann entsprechende Referenzwerte hinzugezogen.
Die Qualität des entwickelten Modells wird durch die Angabe eines Korrelationskoeffizienten R² und eines Analysenfehlers der Kreuzvalidierung RMSEP als statistische Leistungsparameter beschrieben.

Als Datenvorbehandlung wurde für die Erstellung des Kalibrationsmodells in diesem Beispiel die 1. Ableitung nach Norris und ein reduzierter spektraler Bereich von 5022 cm⁻¹ bis 4020 cm⁻¹ genutzt.

Die PLS Kalibration erreicht in der Kreuzvalidierung folgende Kenngrößen: einen Korrelationskoeffizienten von R² = 99,3 % und einen RMSEP-Wert von im Mittel 0,329 bei 8 Faktoren mit der 1. Ableitung nach Norris als Datenvorbehandlung im spektralen Bereich von 5022 cm⁻¹ bis 4020 cm⁻¹.

Um die Güte der Methode durch externe Validierung zu testen, wurden "unabhängige", nicht einkalibrierte Spektren von Testproben mit den Kalibrationsmodellen vorhergesagt und mit den entsprechenden mittels eines unabhängigen analytischen Verfahrens (HPLC) ermittelten Referenzwerten verglichen.

Die Ergebnisse sind in Tabelle 300 dargestellt.

Eine Standardabweichung (Root Mean Square Error of Deviation, RMSED) beträgt in diesem Beispiel 0,34 Gew.%.

Wie in diesem Beispiel gezeigt ist, können Cysteinsäuregehalte neuer Haarmuster somit nach Modellerstellung mittels NIR-Messungen mit den beschriebenen Präzisionen bestimmt werden.

In verschiedenen Ausführungsbeispielen könnte das beschriebene Verfahren alternativ oder ergänzend zum NIR-Bereich mittels des IR-Bereichs ausgeführt werden.

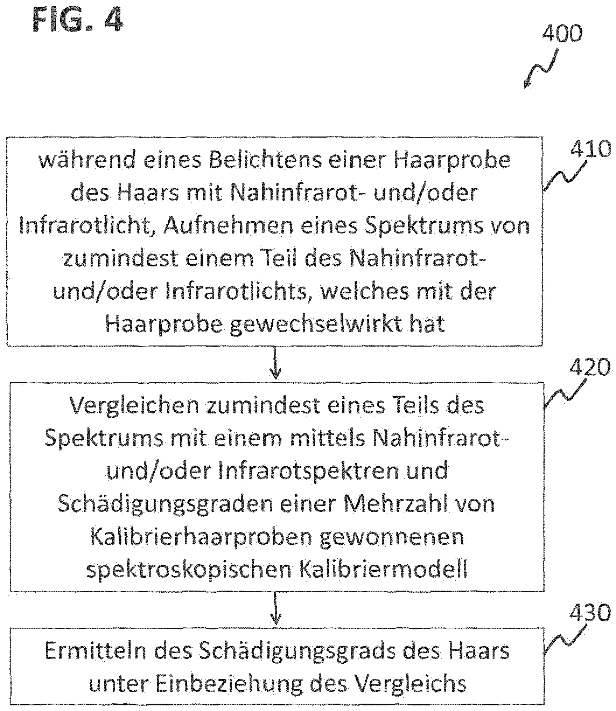

FIG. 4 zeigt ein Ablaufdiagramm 400, welches ein Verfahren zum Ermitteln eines Schädigungsgrads von Haar gemäß verschiedenen Ausführungsbeispielen darstellt.

In verschiedenen Ausführungsbeispielen kann das Verfahren aufweisen während eines Belichtens einer Haarprobe des Haars mit Nahinfrarot- und/oder Infrarotlicht, Aufnehmen eines Spektrums von zumindest einem Teil des Nahinfrarot- und/oder Infrarotlichts, welches mit der Haarprobe gewechselwirkt hat (bei 410), Vergleichen zumindest eines Teils des Spektrums mit einem mittels Nahinfrarot- und/oder Infrarotspektren und Schädigungsgraden einer Mehrzahl von Kalibrierhaarproben gewonnenen spektroskopischen Kalibriermodell (bei 420) und Ermitteln des Schädigungsgrads des Haars unter Einbeziehung des Vergleichs (bei 430).

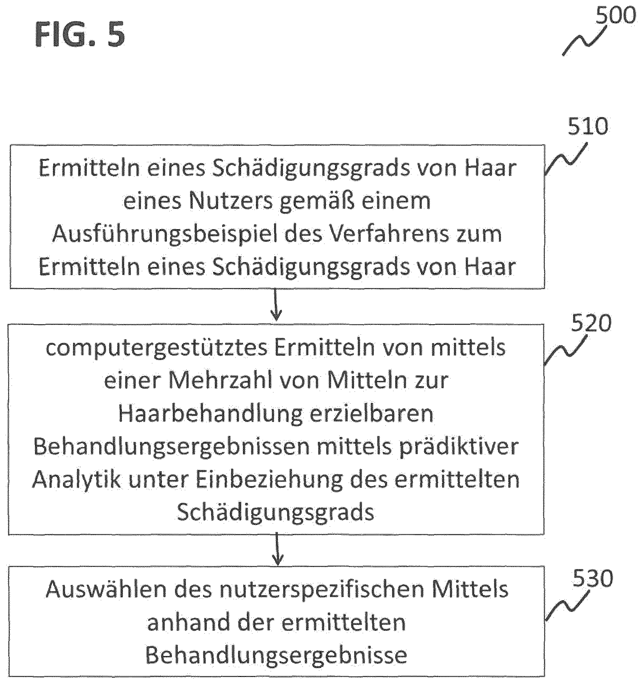

FIG. 5 zeigt ein Ablaufdiagramm 500, welches ein Verfahren zum Ermitteln eines nutzerspezifischen Mittels zur Haarbehandlung gemäß verschiedenen Ausführungsbeispielen darstellt.

In verschiedenen Ausführungsbeispielen kann das oben beschriebene Verfahren zum Ermitteln eines Schädigungsgrads von Haar bzw. die oben beschriebene Vorrichtung zum Ermitteln eines Schädigungsgrads von Haar genutzt werden für ein Verfahren zum Ermitteln eines nutzerspezifischen Mittels zur Haarbehandlung.

In verschiedenen Ausführungsbeispielen kann das Verfahren zum Ermitteln eines nutzerspezifischen Mittels zur Haarbehandlung aufweisen: ein Ermitteln eines Schädigungsgrads von Haar eines Nutzers gemäß einer der oben beschriebenen Ausführungsbeispiele (bei 510), ein computergestütztes Ermitteln von mittels einer Mehrzahl von Mitteln zur Haarbehandlung erzielbaren Behandlungsergebnissen mittels prädiktiver Analytik unter Einbeziehung des ermittelten Schädigungsgrads (bei 520), und ein Auswählen des nutzerspezifischen Mittels anhand der ermittelten Behandlungsergebnisse (bei 530).

Prädiktive Analytik kann allgemein als ein Verfahren beschrieben werden, um aus großen Datenmengen Informationen zu extrahieren und aus diesen Daten ein Modell zu erzeugen, welches es erlaubt, auch für Werte, die nicht Teil des Datensatzes sind, Vorhersagen zu treffen. Bei Anwendung eines Prädiktive Analytik Verfahrens kann typischerweise ein Teil des Datensatzes als Trainings-Datensatz (auch als Trainingssatz oder Trainingsdaten bezeichnet) genutzt werden. Anhand dieses Trainingsdatensatzes können ein oder mehrere Modelle erzeugt werden, welche dann anhand der Daten, die nicht Teil des Trainingsdatensatzes sind, anhand der gesamten Daten, oder anhand eines speziell ausgewählten Teils der Daten, getestet werden können.

Für eine Bewertung des Modells, also eine Ermittlung der Anpassungsgüte, können beispielsweise ein Bestimmtheitsmaß R², ein mittlerer absoluter Fehler, ein mittlerer quadratischer Fehler, eine Standardabweichung und/oder eine mittlere Abweichung herangezogen werden.

Das Bestimmtheitsmaß R² kann für ein lineares Regressionsmodell einem quadrierten Korrelationskoeffizienten entsprechen. Für ein anderes Modell (eine andere Beziehung) kann es anders definiert sein.

Für die Modellierung mittels Prädiktiver Analytik können gemäß verschiedenen Ausführungsbeispielen verschiedene Funktionen oder Verfahren herangezogen werden. In einem einfachen Fall kann beispielsweise eine multiple lineare Regression genutzt werden. Bessere Ergebnisse können typischerweise unter Verwendung von polynomen Regressionen, neuronalen Netzen, Support Vector Machines, Entscheidungsbäumen (beispielsweise Baum-Ensembles) oder ähnlichem erzielt werden.

In verschiedenen Ausführungsbeispielen kann das Verfahren zum Ermitteln eines nutzerspezifischen Mittels zur Haarbehandlung als unabhängigen Parameter für die Modellierung mittels der Prädiktiven Analytik einen gemäß einem der in verschiedenen Ausführungsbeispielen beschriebenen Verfahren zum Ermitteln eines Schädigungsgrads von Haar bestimmten Schädigungsgrad des Haars des Nutzers verwenden. Darüber hinaus können weitere unabhängige Parameter, z.B. eine Grundhaarfarbe des Nutzers, ein Ergrauungsgrad, usw., und als abhängiger Parameter beispielsweise ein gewünschtes Behandlungsergebnis, z.B. eine Wunschhaarfarbe und/oder Wunschhaarstruktur, usw. einbezogen werden.

Anhand dieser Parameter kann mittels der Prädiktiven Analytik wie oben beschrieben ein nutzerspezifische Mittel zur Haarbehandlung ermittelt werden, welches unter Berücksichtigung der unabhängigen Parameter den abhängigen Parameter, z.B. das gewünschte Behandlungsergebnis, erzielt oder diesem zumindest möglichst nahe kommt.

In verschiedenen Ausführungsbeispielen kann das nutzerspezifische Mittel zur Haarbehandlung ein Haarpflegeprodukt, ein Haarfärbeprodukt, ein Blondierungsprodukt, oder ein Stylingprodukt, beispielsweise ein Glättungsmittel wie z.B. Straightener oder Relaxer oder ein Mittel für eine Dauerwelle, aufweisen.

Weitere vorteilhafte Ausgestaltungen des Verfahrens ergeben sich aus der Beschreibung der Vorrichtung und umgekehrt.

## Patentansprüche

1. Verfahren zum Ermitteln eines Schädigungsgrads von Haar, aufweisend:
während eines Belichtens einer Haarprobe des Haars mit Nahinfrarot- und/oder Infrarotlicht, Aufnehmen eines Spektrums von zumindest einem Teil des Nahinfrarot- und/oder Infrarotlichts, welches mit der Haarprobe gewechselwirkt hat;
Vergleichen zumindest eines Teils des Spektrums mit einem mittels Nahinfrarot- und/oder Infrarotspektren und Schädigungsgraden einer Mehrzahl von Kalibrierhaarproben gewonnenen spektroskopischen Kalibriermodell; und
Ermitteln des Schädigungsgrads des Haars unter Einbeziehung des Vergleichs;
Erstellen des Kalibriermodells, aufweisend:
für die Mehrzahl von Kalibrierhaarproben:
während eines Belichtens der Kalibrierhaarprobe mit Nahinfrarot- und/oder Infrarotlicht, Aufnehmen eines Kalibrierspektrums von zumindest einem Teil des Nahinfrarot- und/oder Infrarotlichts, welches mit der Kalibrierhaarprobe gewechselwirkt hat;
Ermitteln eines Schädigungsgrads der Kalibrierhaarprobe mittels eines unabhängigen analytischen Verfahrens; und
Zuweisen des Schädigungsgrads zum Kalibrierspektrum; und
Ermitteln einer Korrelation zwischen der Mehrzahl von Kalibrierspektren und der Mehrzahl von Schädigungsgraden,
wobei das analytische Verfahren ein Ermitteln eines Cysteinsäuregehalts aufweist.

2. Verfahren gemäß Anspruch 1,
wobei das Ermitteln des Cysteinsäuregehalts ein chromatographisches Verfahren aufweist.

3. Verfahren gemäß einem der Ansprüche 1 oder 2,
wobei das Ermitteln einer Korrelation zwischen der Mehrzahl von Kalibrierspektren und der Mehrzahl von Schädigungsgraden ein Anwenden eines Partial Least Squares-Algorithmus aufweist.

4. Verfahren gemäß einem der Ansprüche 1 bis 3,
wobei der zumindest eine Teil des Nahinfrarot- und/oder Infrarotlichts einen Wellenzahlbereich von 5022 cm⁻¹ bis 4020 cm⁻¹ aufweist.

5. Verfahren gemäß einem der Ansprüche 1 bis 4,
wobei der zumindest eine Teil des Nahinfrarot- und/oder Infrarotlichts einen Wellenzahlbereich von 12.820 cm⁻¹ bis 7692 cm⁻¹ aufweist.

6. Vorrichtung zum Ermitteln eines Schädigungsgrads von Haar mittels eines Verfahrens gemäß einem der Ansprüche 1 bis 5, aufweisend:
eine NIR-Lichtquelle oder/und eine IR-Lichtquelle zum Belichten einer Haarprobe des Haars mit NIR- bzw. IR-Licht;
ein Spektrometer zum Aufnehmen eines Spektrums von zumindest einem Teil des NIR- bzw. IR-Lichts, welches mit der Haarprobe gewechselwirkt hat; und
eine Datenverarbeitungsvorrichtung mit einem Datenspeicher, in welchem ein mittels Nahinfrarot- und/oder Infrarotspektren und Schädigungsgraden einer Mehrzahl von Kalibrierhaarproben gewonnenes spektroskopisches Kalibriermodell gespeichert ist, und mit einem Prozessor zum Vergleichen zumindest eines Teils des Spektrums mit dem spektroskopischen Kalibriermodell und zum Ermitteln des Schädigungsgrads des Haars unter Einbeziehung des Vergleichs,
wobei die Datenverarbeitungsvorrichtung dazu eingerichtet ist, das Verfahren gemäß einem der Ansprüche 1 bis 5 auszuführen.

7. Vorrichtung gemäß Anspruch 6,
wobei die NIR- bzw. IR-Lichtquelle und das Spektrometer eine integrierte Einheit bilden.

8. Vorrichtung gemäß einem der Ansprüche 6 oder 7,
wobei der zumindest eine Teil des Nahinfrarot- und/oder Infrarotlichts einen Wellenzahlbereich von 5022 cm⁻¹ bis 4020 cm⁻¹ aufweist.

9. Vorrichtung gemäß einem der Ansprüche 6 oder 7,
wobei der zumindest eine Teil des Nahinfrarot- und/oder Infrarotlichts einen Wellenzahlbereich von 12.820 cm⁻¹ bis 7692 cm⁻¹ aufweist.

10. Vorrichtung gemäß einem der Ansprüche 6 bis 9,
wobei die integrierte Einheit und/oder die Datenverarbeitungsvorrichtung mobile Geräte sind.

11. Vorrichtung gemäß einem der Ansprüche 6 bis 10,
wobei die Datenverarbeitungsvorrichtung ferner eingerichtet ist, in Abhängigkeit vom ermittelten Schädigungsgrad eine individuelle Haarbehandlungsanweisung zu ermitteln.

12. Verfahren zum Ermitteln eines nutzerspezifischen Mittels zur Haarbehandlung, aufweisend: Ermitteln eines Schädigungsgrads von Haar eines Nutzers gemäß einem der Ansprüche 1 bis 5;
computergestütztes Ermitteln von mittels einer Mehrzahl von Mitteln zur Haarbehandlung erzielbaren Behandlungsergebnissen mittels prädiktiver Analytik unter Einbeziehung des ermittelten Schädigungsgrads; und
Auswählen des nutzerspezifischen Mittels anhand der ermittelten Behandlungsergebnisse.

13. Verfahren gemäß Anspruch 12,
wobei das nutzerspezifische Mittel zur Haarbehandlung ein Haarpflegeprodukt oder ein Haarfärbeprodukt, ein Blondierungsprodukt, oder ein Stylingprodukt, beispielsweise ein Glättungsmittel, insbesondere einen Straightener oder Relaxer, oder ein Mittel für eine Dauerwelle, aufweist.

## Claims

1. A method for determining a degree of damage to hair, comprising:
while exposing a hair sample of the hair to near-infrared and/or infrared light, recording a spectrum of at least one part of the near-infrared and/or infrared light that has interacted with the hair sample;
comparing at least one part of the spectrum with a spectroscopic calibration model obtained by means of near-infrared and/or infrared spectra and degrees of damage to a plurality of calibration hair samples; and
determining the degree of damage to the hair, taking into account the comparison;
creating the calibration model, comprising:
for the plurality of calibration hair samples:
while exposing the calibration hair sample to near-infrared and/or infrared light,
recording a calibration spectrum of at least one part of the near-infrared and/or
infrared light that has interacted with the calibration hair sample;
determining a degree of damage to the calibration hair sample using an independent analytical method; and
assigning the degree of damage to the calibration spectrum; and
determining a correlation between the plurality of calibration spectra and the plurality of degrees of damage, wherein the analytical method comprises determining a cysteic acid content.

2. The method according to claim 1, wherein determining the cysteic acid content comprises a chromatographic method.

3. The method according to one of claims 1 or 2, wherein determining a correlation between the plurality of calibration spectra and the plurality of degrees of damage comprises employing a partial least squares algorithm.

4. The method according to one of claims 1 to 3, wherein the at least one part of the near-infrared and/or infrared light has a wave number range of from 5022 cm⁻¹ to 4020 cm⁻¹.

5. The method according to one of claims 1 to 4, wherein the at least one part of the near-infrared and/or infrared light has a wave number range of from 12,820 cm⁻¹ to 7692 cm⁻¹.

6. A device for determining a degree of damage to hair by means of a method according to one of claims 1 to 5, comprising:
an NIR light source and/or an IR light source for exposing a hair sample of the hair to NIR or IR light;
a spectrometer for recording a spectrum of at least one part of the NIR or IR light that has interacted with the hair sample; and
a data processing device having a data memory in which a spectroscopic calibration model obtained by means of near-infrared and/or infrared spectra and degrees of damage to a plurality of calibration hair samples is stored, and having a processor for comparing at least one part of the spectrum with the spectroscopic calibration model and for determining the degree of damage to the hair, taking into account the comparison,
wherein the data processing device is designed to carry out the method according to one of claims 1 to 5.

7. The device according to claim 6, wherein the NIR or IR light source and the spectrometer form an integrated unit.

8. The device according to one of claims 6 or 7, wherein the at least one part of the near-infrared and/or infrared light has a wave number range of from 5022 cm⁻¹ to 4020 cm⁻¹.

9. The device according to one of claims 6 or 7, wherein the at least one part of the near-infrared and/or infrared light has a wave number range of from 12,820 cm⁻¹ to 7692 cm⁻¹.

10. The device according to one of claims 6 to 9, wherein the integrated unit and/or the data processing device are mobile devices.

11. The device according to one of claims 6 to 10, wherein the data processing device is further designed to determine individual hair treatment instructions depending on the determined degree of damage.

12. A method for determining a user-specific agent for hair treatment, comprising:
determining a degree of damage to the hair of a user according to one of claims 1 to 5;
determining, in a computerized manner, treatment results that can be achieved by means of a plurality of agents for hair treatment by means of predictive analysis, taking into account the determined degree of damage; and
selecting the user-specific agent based on the determined treatment results.

13. The method according to claim 12, wherein the user-specific agent for hair treatment comprises a hair care product or a hair coloring product, a bleaching product, or a styling product, for example a straightening agent, in particular a straightener or relaxer, or an agent for a perm.

## Revendications

1. Procédé permettant de déterminer un degré de détérioration des cheveux, comprenant :
l'enregistrement d'un spectre d'au moins une partie de la lumière infrarouge proche et/ou de la lumière infrarouge qui a interagi avec un échantillon de cheveux pendant l'exposition de l'échantillon de cheveux à une lumière infrarouge proche et/ou à une lumière infrarouge ;
la comparaison d'au moins une partie du spectre à un modèle d'étalonnage spectroscopique obtenu au moyen de spectres infrarouges proches et/ou infrarouges et de degrés de détérioration d'une pluralité d'échantillons de cheveux d'étalonnage ; et
la détermination du degré de détérioration des cheveux en tenant compte de la comparaison ;
la création du modèle d'étalonnage, comprenant :
pour la pluralité d'échantillons de cheveux d'étalonnage :
l'enregistrement d'un spectre d'étalonnage d'au moins une partie de la lumière infrarouge proche et/ou de la lumière infrarouge qui a interagi avec un échantillon de cheveux d'étalonnage pendant l'exposition de l'échantillon de cheveux d'étalonnage à une lumière infrarouge proche et/ou à une lumière infrarouge ;
la détermination du degré de détérioration de l'échantillon de cheveux d'étalonnage au moyen d'un procédé analytique indépendant ; et
l'attribution du degré de détérioration au spectre d'étalonnage ; et
la détermination d'une corrélation entre la pluralité de spectres d'étalonnage et la pluralité de degrés de détérioration,
le procédé analytique comprenant la détermination d'une teneur en acide cystéique.

2. Procédé selon la revendication 1,
dans lequel la détermination de la teneur en acide cystéique comprend un procédé chromatographique.

3. Procédé selon l'une des revendications 1 ou 2,
dans lequel la détermination d'une corrélation entre la pluralité de spectres d'étalonnage et la pluralité de degrés de détérioration comprend l'application d'un algorithme des moindres carrés partiels.

4. Procédé selon l'une des revendications 1 à 3,
dans lequel l'au moins une partie de la lumière infrarouge proche et/ou de la lumière infrarouge comprend une plage de nombre d'ondes de 5 022 cm⁻¹ à 4 020 cm⁻¹.

5. Procédé selon l'une des revendications 1 à 4,
dans lequel l'au moins une partie de la lumière infrarouge proche et/ou de la lumière infrarouge comprend une plage de nombre d'ondes de 12 820 cm⁻¹ à 7 692 cm⁻¹.

6. Dispositif de détermination du degré de détérioration des cheveux au moyen d'un procédé selon l'une des revendications 1 à 5, comprenant :
une source de lumière NIR et/ou une source de lumière IR destinées à exposer un échantillon de cheveux à la lumière NIR ou IR ;
un spectromètre destiné à enregistrer un spectre d'au moins une partie de la lumière NIR ou IR qui a interagi avec l'échantillon de cheveux ; et
un dispositif de traitement de données comportant une mémoire de données dans laquelle est stocké un modèle d'étalonnage spectroscopique obtenu au moyen de spectres infrarouges proches et/ou infrarouges et de degrés de détérioration d'une pluralité d'échantillons de cheveux d'étalonnage, et un processeur destiné à comparer au moins une partie du spectre au modèle d'étalonnage spectroscopique et à déterminer le degré de détérioration des cheveux en tenant compte de la comparaison, le dispositif de traitement de données étant configuré pour mettre en œuvre le procédé selon l'une des revendications 1 à 5.

7. Dispositif selon la revendication 6,
dans lequel la source de lumière NIR ou IR et le spectromètre forment une unité intégrée.

8. Dispositif selon l'une des revendications 6 ou 7,
dans lequel l'au moins une partie de la lumière infrarouge proche et/ou de la lumière infrarouge présente une plage de nombre d'ondes de 5 022 cm⁻¹ à 4 020 cm⁻¹.

9. Dispositif selon l'une des revendications 6 ou 7,
dans lequel l'au moins une partie de la lumière infrarouge proche et/ou de la lumière infrarouge présente une plage de nombre d'ondes de 12 820 cm⁻¹ à 7 692 cm⁻¹.

10. Dispositif selon l'une des revendications 6 à 9,
dans lequel l'unité intégrée et/ou le dispositif de traitement de données sont des appareils mobiles.

11. Dispositif selon l'une des revendications 6 à 10,
dans lequel le dispositif de traitement de données est en outre configuré pour déterminer une instruction de traitement de cheveux individuelle en fonction du degré de détérioration déterminé.

12. Procédé permettant de déterminer un agent spécifique à l'utilisateur destiné au traitement des cheveux, comprenant :
la détermination d'un degré de détérioration des cheveux d'un utilisateur selon l'une des revendications 1 à 5 ;
la détermination assistée par ordinateur des résultats de traitement pouvant être obtenus au moyen d'une pluralité de moyens destinés au traitement des cheveux au moyen d'une analyse prédictive en tenant compte du degré de détérioration déterminé ; et
la sélection de l'agent spécifique à l'utilisateur sur la base des résultats de traitement déterminés.

13. Procédé selon la revendication 12,
dans lequel l'agent spécifique à l'utilisateur destiné au traitement des cheveux comprend un produit de soin des cheveux ou un produit de coloration des cheveux, un produit de décoloration ou un produit de coiffage, par exemple un agent de lissage, en particulier un lisseur ou un défrisant, ou un agent pour permanente.
